# EUROPEAN PATENT APPLICATION

(11) **EP 2 586 364 A1**
(43) Date of publication of application: **01.05.2013**
(21) Application number: 12190094.8
(22) Date of filing: 26.10.2012
(51) Int. Cl.: A61B 5/00, A61B 5/107, G01B 11/25

(54) **Point size light illumination in metrology systems for in-situ surgical applications**

(30) Priority: 27.10.2011 US 201161551961 P; 05.10.2012 US 201213645559
(71) Applicant: Covidien LP, Mansfield, MA 02048 (US)
(72) Inventor: Sharonov, Alexey, Bethany, Connecticut 06524 (US)
(74) Representative: Soames, Candida Jane

(57) **Abstract**

A metrology method includes the steps of positioning a point source projector at a known distance from a target site, projecting a light beam through a semi-transparent mask, forming a magnified pattern on the target site from the light beam, visually inspecting a portion of the magnified pattern that is formed on the target site, and determining a measurement of the target site based on the known dimensions of the mask pattern, a magnification factor, and the portion of the magnified pattern that is formed on the target site. The light beam diverges from a point and the semi-transparent mask has a mask pattern of known dimensions. The magnified pattern is magnified from the mask pattern by the magnification factor.

## Description

### CROSS REFERENCE TO RELATED APPLICATION

The present application claims the benefit of and priority to U.S. Provisional Application Serial No.61/551,961, filed on October 27, 2011, the entire contents of which are incorporated herein by reference.

### BACKGROUND

### 1. Technical Field

The present disclosure relates to a method for measuring a dimension of a target site. More particularly, the present disclosure relates to a method of projecting an image for use in measuring a dimension of a target site.

### 2. Background of the Related Art

Minimally invasive surgery, e.g., laparoscopic, endoscopic, and thoroscopic surgery, has many advantages over traditional open surgeries. In particular, minimally invasive surgery eliminates the need for a large incision, thereby reducing discomfort, recovery time, and many of the deleterious side effects associated with traditional open surgery.

The minimally invasive surgeries are performed through small openings in a patient's skin. These openings may be incisions in the skin or may be naturally occurring body orifices (e.g., mouth, anus, or vagina). In general, insufflation gas is used to enlarge the area surrounding the target surgical site to create a larger, more accessible work area.

During minimally invasive procedures, it is often difficult for a surgeon to determine sizes of various organs, tissues, and other structures in a surgical site. Various in-situ surgical metrology methods exist for measurement in a surgical site. Such methods require many moving parts and projection images that change size and/or focus quickly as projectors move in or out of a surface of projection. A continuing need exists for in-situ surgical metrology methods that operate with a stable focus and no moving parts.

### SUMMARY

A metrology method includes the steps of positioning a point source projector at a known distance from a target site, projecting a light beam through a semi-transparent mask, forming a magnified pattern on the target site from the light beam, visually inspecting a portion of the magnified pattern that is formed on the target site, and determining a measurement of the target site based on the known dimensions of the mask pattern, a magnification factor, and the portion of the magnified pattern that is formed on the target site. The light beam diverges from a point and the semi-transparent mask has a mask pattern of known dimensions. The magnified pattern is magnified from the mask pattern by the magnification factor.

The light beam may be a laser emitted by a laser diode disposed within the point source projector. The light beam may be emitted by an LED disposed within the point source projector. The light beam may be focused to the point by a lens disposed within the point source projector. The semi-transparent mask may be translatable to adjust the magnification factor. The semi-transparent mask may be disposed within the point source projector. The point source projector may be attached to an endoscope for visually inspecting the target site. The mask pattern may include a series of uniformly spaced concentric circles. The mask pattern may also or alternatively include a series of uniformly spaced linear markings.

### BRIEF DESCRIPTION OF THE DRAWINGS

The above and other aspects, features, and advantages of the present disclosure will become more apparent in light of the following detailed description when taken in conjunction with the accompanying drawings in which:

**FIG. 1** is a side, schematic view of a metrology system according to the principles of the present disclosure;

**FIG. 2** is a side, perspective view of a method of use of the metrology system of FIG. 1; and

**FIG. 3** is a side, schematic view of a metrology system according to another embodiment of the present disclosure.

### DETAILED DESCRIPTION

Particular embodiments of the present disclosure are described hereinbelow with reference to the accompanying drawings; however, it is to be understood that the disclosed embodiments are merely exemplary of the disclosure and may be embodied in various forms. Well-known functions or constructions are not described in detail to avoid obscuring the present disclosure in unnecessary detail. Therefore, specific structural and functional details disclosed herein are not to be interpreted as limiting, but merely as a basis for the claims and as a representative basis for teaching one skilled in the art to variously employ the present disclosure in virtually any appropriately detailed structure.

Like reference numerals may refer to similar or identical elements throughout the description of the figures. As shown in the drawings and described throughout the following description, as is traditional when referring to relative positioning on a surgical instrument, the term "proximal" refers to the end of the apparatus which is closer to the user and the term "distal" refers to the end of the apparatus which is farther away from the user. The term "clinician" refers to any medical professional (i.e., doctor, surgeon, nurse, or the like) performing a medical procedure involving the use of embodiments described herein.

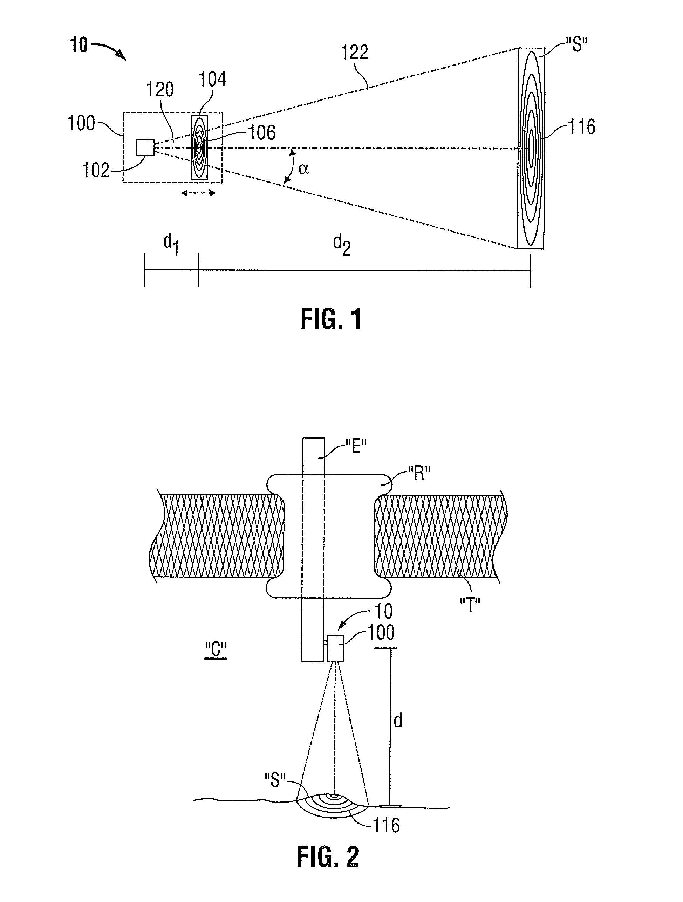

As seen in **FIG. 1****,** a metrology system **10** includes a point source projector **100** having a point source light emitter **102** and a mask **104.** Mask **104** is a distance **d₁** from point source light emitter **102** and a distance **d₂** from a target site **"S".** Mask **104** is semi-transparent and has a substantially opaque mask pattern **106** thereon. Mask pattern **106** has markings of known distances therebetween. For example, mask pattern **106** may be a series of uniformly spaced concentric circles. Mask **104** may be translatable toward or away from point source light emitter **102.**

Point source light emitter **102** emits a light beam **120** therefrom. Light beam **120** approximates a point at point source light emitter **102** and conically diverges therefrom at an angle α. Point source light emitter **102** may be any device capable of emitting light from a narrow point, such as a laser diode or an LED. Light beam **120** is partially blocked by mask pattern **106** upon incidence with mask **104.** An unblocked portion **122** of light beam **120** continues past mask **104** to reach target site **"S".** Unblocked portion **122** creates a magnified pattern **116** on target site **"S".** Magnified pattern **116** is magnified from mask pattern **106** according to a formula: **M** = **1** + **d₂/d₁,** where **M** is a magnification factor between mask pattern **106** and magnified pattern **116.** A translation of mask **104** or point source projector **100** away from target site **"S"** increases magnification factor **M.** A translation of mask **104** or point source projector **100** toward target site **"S"** decreases magnification factor **M.** Magnified pattern **116** retains a substantially sharp focus as mask **104** and/or point source projector **100** is translated.

A method of use of metrology system **10** is depicted in **FIG. 2****.** Metrology system 10 is attached to a distal end of an endoscope **"E".** Endoscope **"E"** is inserted into a body cavity **"C"** through an opening in a tissue **"T".** Endoscope **"E"** may be inserted through a seal anchor **"R"** positioned within the opening in tissue **"T".** Endoscope **"E"** is inserted through a port in seal anchor **"R"** that is expanded to a width greater than a maximum combined width of endoscope **"E"** and point source projector **100.** Once the distal end of endoscope **"E"** is distal to seal anchor **"R",** the port resiliently compresses to form a substantially airtight seal around endoscope **"E".** Point source projector **100** is translated distally toward target site **"S"** until point source projector **100** arrives at a known distance **d** from target site **"S".** The arrival of point source projector **100** at distance **d** may be determined through any appropriate means, such as triangulation. Distance **d₁** may be fixed prior to insertion of endoscope **"E".** Alternatively, endoscope **"E"** may include a mechanism, such as a rotatable knob (not shown), for altering distance **d₁.** Distance **d₂** is calculated by subtracting distance **d₁** from distance **d.** Distance **d₁** and distance **d₂** may then be used to calculate magnification factor **M.**

Point source projector **100** projects magnified pattern **116** onto target site **"S".** A clinician may observe magnified pattern **116** through endoscope **"E".** A dimension of target site **"S"** is measured by visually inspecting and counting a number **n** of uniformly spaced markings appearing along the dimension of target site **"S".** The number **n** of uniformly spaced markings is multiplied by a uniform distance between individual markings of pattern **116.** The uniform distance between individual markings of pattern **116** is calculated by multiplying a uniform distance **dₖ** between individual markings of mask **104** by magnification factor **M.** Thus, a measure of the dimension of target site **"S"** is calculated according to the formula: **x** = **nMdₖ,** where x is the measure of the dimension.

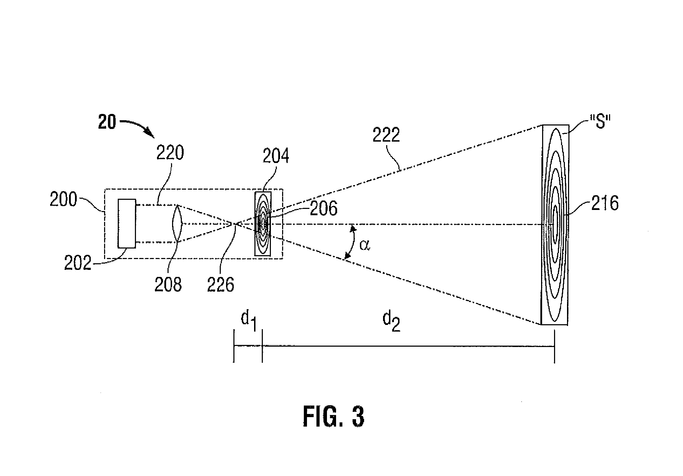

Turning to **FIG. 3****,** a metrology system in accordance with an alternate embodiment of the present disclosure is generally designated as **20.** Metrology system **20** is similar to metrology system **10** and thus will only be discussed as necessary to identify the differences in construction and operation thereof.

Metrology system **20** includes a point source projector **200** having a light source **202,** a mask **204,** and a lens **208.** Mask **204** has a mask pattern **206.** Light source **202** emits a light beam **220** toward lens **208.** Lens **208** is a converging lens that focuses light beam **220** into a point **226.** Point **226** is a distance **d₁** away from mask **204.** Light beam **220** diverges at an angle α from point **226** and is partially blocked by mask **204.** An unblocked beam **222** passes through mask **204** and travels a distance **d₂** to a target site "S" to form a magnified pattern **216** thereon.

A method of use of metrology system **20** is substantially identical to the method of use of metrology system **10** described hereinabove.

It should be understood that the foregoing description is only illustrative of the present disclosure. Various alternatives and modifications can be devised by those skilled in the art without departing from the disclosure. Accordingly, the present disclosure is intended to embrace all such alternatives, modifications and variances. The embodiments described with reference to the attached drawing figs. are presented only to demonstrate certain examples of the disclosure. Other elements, steps, methods and techniques that are insubstantially different from those described above and/or in the appended claims are also intended to be within the scope of the disclosure.

The invention can be described by reference to the following numbered paragraphs:-
1. A metrology method, comprising the steps of:
   positioning a point source projector at a known distance from a target site;
   projecting a light beam through a semi-transparent mask, wherein the light beam diverges from a point and the semi-transparent mask has a mask pattern of known dimensions;
   forming a magnified pattern on the target site from the light beam, wherein the magnified pattern is magnified from the mask pattern by a magnification factor;
   visually inspecting a portion of the magnified pattern that is formed on the target site; and
   determining a measurement of the target site based on the known dimensions of the mask pattern, the magnification factor, and the portion of the magnified pattern that is formed on the target site.
2. A metrology method according to paragraph 1, wherein the light beam is a laser emitted by a laser diode disposed within the point source projector.
3. A metrology method according to paragraph 1, wherein the light beam is emitted by an LED disposed within the point source projector.
4. A metrology method according to paragraph 1, wherein the light beam is focused to the point by a lens disposed within the point source projector.
5. A metrology method according to paragraph 1, wherein the semi-transparent mask is translatable to adjust the magnification factor.
6. A metrology method as in paragraph 1, wherein the semi-transparent mask is disposed within the point source projector.
7. A metrology method according to paragraph 1, wherein the point source projector is attached to an endoscope for visually inspecting the target site.
8. A metrology method according to paragraph 1, wherein the mask pattern includes a series of uniformly spaced concentric circles.
9. A metrology method according to paragraph 1, wherein the mask pattern includes a series of uniformly spaced linear markings.

## Claims

1. A metrology method, comprising the steps of:
positioning a point source projector at a known distance from a target site;
projecting a light beam through a semi-transparent mask, wherein the light beam diverges from a point and the semi-transparent mask has a mask pattern of known dimensions;
forming a magnified pattern on the target site from the light beam, wherein the magnified pattern is magnified from the mask pattern by a magnification factor;
visually inspecting a portion of the magnified pattern that is formed on the target site; and
determining a measurement of the target site based on the known dimensions of the mask pattern, the magnification factor, and the portion of the magnified pattern that is formed on the target site.

2. A metrology method according to claim 1, wherein the light beam is a laser emitted by a laser diode disposed within the point source projector.

3. A metrology method according to claim 1, wherein the light beam is emitted by an LED disposed within the point source projector.

4. A metrology method according to any preceding claims, wherein the light beam is focused to the point by a lens disposed within the point source projector.

5. A metrology method according to any preceding claims, wherein the semi-transparent mask is translatable to adjust the magnification factor.

6. A metrology method as in any preceding claim, wherein the semi-transparent mask is disposed within the point source projector.

7. A metrology method according to any preceding claim, wherein the point source projector is attached to an endoscope for visually inspecting the target site.

8. A metrology method according to any preceding claim, wherein the mask pattern includes a series of uniformly spaced concentric circles.

9. A metrology method according to any of claims 1 to 8, wherein the mask pattern includes a series of uniformly spaced linear markings.
